# EUROPEAN PATENT SPECIFICATION

(11) **EP 3 395 943 B1**
(45) Date of publication and mention of the grant of the patent: **30.11.2022**
(21) Application number: 16878613.5
(22) Date of filing: 19.12.2016
(51) Int. Cl.: C12N 5/077, C12N 5/0775, C12Q 1/02, C12N 5/071, C12N 5/00, C12N 5/0789, C12N 5/074

(54) **METHOD FOR MANUFACTURING TISSUE/ORGAN BY USING BLOOD CELLS**
VERFAHREN ZUR HERSTELLUNG VON GEWEBE/ORGANEN MITTELS BLUTZELLEN
PROCÉDÉ DE FABRICATION D'UN TISSU/ORGANE EN UTILISANT DES CELLULES SANGUINES

(30) Priority: 24.12.2015 JP 2015251140
(43) Date of publication of application: 31.10.2018
(73) Proprietor: Public University Corporation Yokohama City University, Yokohama-shi, Kanagawa 236-0027 (JP)
(72) Inventor: TANIGUCHI, Hideki, Yokohama-shi Kanagawa 236-0004 (JP); NIE, Yunzhong, Yokohama-shi Kanagawa 236-0004 (JP); ZHENG, Yun-Wen, Yokohama-shi Kanagawa 236-0004 (JP); SEKINE, Keisuke, Yokohama-shi Kanagawa 236-0004 (JP); TAKEBE, Takanori, Yokohama-shi Kanagawa 236-0004 (JP)
(74) Representative: J A Kemp LLP
(86) International application number: PCT/JP2016/087739
(87) International publication number: WO 2017/110724

(56) References cited:
- WO-A1-2013/047639
- WO-A1-2015/012158
- TAKANORI TAKEBE ET AL: "Vascularized and Complex Organ Buds from Diverse Tissues via Mesenchymal Cell-Driven Condensation", CELL STEM CELL, vol. 16, no. 5, 7 May 2015 (2015-05-07), pages 556-565, XP055329771, AMSTERDAM, NL ISSN: 1934-5909, DOI: 10.1016/j.stem.2015.03.004
- T TAKEBE ET AL: "Human iPSC-Derived Miniature Organs: A Tool for Drug Studies", CLINICAL PHARMACOLOGY & THERAPEUTICS, vol. 96, no. 3, 21 May 2014 (2014-05-21), pages 310-313, XP55173771, ISSN: 0009-9236, DOI: 10.1038/clpt.2014.110
- SHIBASHISH GIRI ET AL: "Preclinical Liver Bud Engineering towards Clinical Target for Liver Diseases", JOURNAL OF CLINICAL AND EXPERIMENTAL HEPATOLOGY, vol. 4, no. 1, March 2014 (2014-03), pages 71-74, XP055595961, ISSN: 0973-6883, DOI: 10.1016/j.jceh.2014.02.151
- TAKEBE, TAKANORI. et al.: "Generation of a vascularized and functional human liver from an iPSC-derived organ bud transplant", NATURE PROTOCOLS, vol. 9, no. 2, 23 January 2014 (2014-01-23), pages 396-409, XP055166485,
- TAKANORI TAKEBE et al.: "Creation of vascularized and functional organ from human iPS cell", Organ Biology, vol. 20, no. 2, 2013, pages 182-185, XP009511496,
- HIDEKI TANIGUCHI et al.: "Generation of a vascularized human liver from an iPSC- derived organ bud transplant", Kan Tan Sui, vol. 70, no. 3, March 2015 (2015-03), pages 353-360, XP009511499,

## Description

### TECHNICAL FIELD

The present invention relates to a method for preparing tissues and organs using blood cells.

### BACKGROUND ART

Recently, methods of generating human functional cells useful for drug discovery screening and regenerative medicine by directed differentiation using pluripotent stem cells, such as iPS cells capable of differentiating into various functional cells, have been attracting attention. To date, the present inventors have established a method of reconstructing human tissues/organs which have well-ordered, three-dimensional structures composed of vascular endothelial cells and mesenchymal cells seen in adult tissues (Non-Patent Document No. 1: Takebe T. et al., Nature (2013); Patent Document No. 1: Method for Preparing Tissue and Organ WO2013/047639 A1).

On the other hand, as a finding concerning the maturing of hematopoietic cells and hepatocytes, reports have been made that a cytokine produced by hematopoietic cells in the developmental process of mouse promotes the maturing of hepatocytes (Non-Patent Document No. 2: Kamiya A., et al., EMBO J. 1999 18:2127-3; Non-Patent Document No. 3: Kinoshita T., et al., Proc Natl Acad Sci U S A. 1999 96:7265-70 (1999)). Although reports have been made that human hepatocytes and human hematopoietic cells are simultaneously engrafted by transplanting a mixture of hematopoietic cells and immature hepatocytes in an immunodeficient animal, no findings have been reported to date regarding the maturity of hepatocytes resulting from simultaneous transplantation with hematopoietic cells (Non-Patent Document No. 4: Bility MT., et al., Nat Protoc. 7:1608-17 (2012); Non-Patent Document No. 5: Washburn ML., et al., Gastroenterology. 140:1334-44 (2011)). Further, no findings have been reported yet that show the importance of hematopoietic cells in the maturing of a functional cell of interest by introducing them into an *in vitro* reconstructed three-dimensional tissue.

### PRIOR ART LITERATURE

### Non-Patent Documents

Non-Patent Document No. 1: Takebe T. et al., Nature (2013)
Non-Patent Document No. 2: Kamiya A., et al., EMBO J. 1999 18:2127-3
Non-Patent Document No. 3: Kinoshita T., et al., Proc Natl Acad Sci U S A. 1999 96:7265-70 (1999)
Non-Patent Document No. 4: Bility MT., et al., Nat Protoc. 7:1608-17 (2012)
Non-Patent Document No. 5: Washburn ML., et al., Gastroenterology. 140:1334-44 (2011)

### Patent Document

Patent Document No. 1: WO2013/047639 A1

### DISCLOSURE OF THE INVENTION

### PROBLEM FOR SOLUTION BY THE INVENTION

When medical application of human tissues/organs prepared by the conventional method is attempted, preparation cost is extremely high; approximately 10 million Yen per adult is needed. This has been a barrier against the spreading of the technology. In view of the current situation that there are at least 20 thousands or more absolute adaptation patients (international estimate of adaptation-exclusion cases/fatal cases in the hepatic failure patients waiting), development of a revised technique which greatly improves function per cell and realizes huge cost reduction is eagerly desired in order to widely adapt such a therapeutic approach to patients as a general purpose technology.

It is an object of the present invention to provide such a revised technique.

### MEANS TO SOLVE THE PROBLEM

The present inventors have succeeded in further improving the function of a resultant organ bud by adding hematopoietic cells to a culture system co-culturing hepatic endoderm cells, vascular endothelial cells and mesenchymal cells. To date, no technique has existed that shows the effect of hematopoietic cells in three-dimensional reconstruction of human tissues/organs. Therefore, it is believed that the method of the present invention is extremely high in novelty and inventiveness.

The invention is defined in the appended set of claims. The gist of the present invention is as described below.
(1) A method of preparing a liver bud, comprising culturing vascular endothelial cells, mesenchymal cells, a cell capable of differentiating into a liver cell, and blood cells, wherein the blood cells are derived from cord blood, *in vitro.*
(2) The method of (1), wherein the liver bud is a structure capable of differentiating into a liver through maturing.
(3) The method of (1) or (2), wherein cells are cultured without using scaffold materials.
(4) The method of any one of (1) to (3), wherein the blood cell comprises an undifferentiated hematopoietic cell, and optionally wherein the undifferentiated hematopoietic cell is a hematopoietic progenitor cell and/or a hematopoietic stem cell.
(5) The method of any one of (1) to (4), wherein the blood cell derived from cord blood is a cell of the monocyte fraction of cord blood.
(6) The method of any one of (1) to (5), wherein the cell capable of differentiating into a liver cell is: (i) an undifferentiated cell induced from a pluripotent stem cell, and optionally wherein the pluripotent stem cell is an induced pluripotent stem cell; or (ii) a hepatic endoderm cell.
(7) The method of (6), wherein the pluripotent stem cell is derived from human.
(8) The method of any one of (1) to (7), wherein a liver bud with an improved albumin secretory capacity is prepared compared with a liver bud prepared by culturing vascular endothelial cells, mesenchymal cells and a cell capable of differentiating into a liver cell *in vitro* in the absence of blood cells.
(9) The method of (8), wherein a liver bud with increased expression of hepatocyte differentiation marker genes is prepared compared with a liver bud prepared by culturing vascular endothelial cells, mesenchymal cells and a cell capable of differentiating into a liver cell *in vitro* in the absence of blood cells.
(10) The method of (9), wherein the hepatocyte differentiation marker gene is at least one marker selected from the group consisting of α fetoprotein, albumin, CYP3A7, tryptophan metabolic enzyme TDO2 and sodium-taurocholate cotransporter.
(11) A liver bud prepared by the method of any one of (1) to (10).
(12) A method of preparing a human liver, comprising preparing a human liver bud by the method of any one of (1) to (10), wherein the liver bud is capable of differentiating into a human liver after the liver bud has been transplanted into a non-human animal.
(13) The liver bud of (11) for use in a therapeutic method comprising transplanting the liver bud into a human or a non-human animal in need thereof.
(14) The liver bud of (11) for use in a therapeutic method of regeneration or function recovery of a liver, comprising transplanting the liver bud into a human or a non-human animal and differentiating the liver bud into a liver.
(15) A method of preparing a non-human chimeric animal, comprising preparing a human liver bud by the method of any of (1) to (10), wherein the liver bud is capable of differentiating into a human liver after it has been transplanted into a non-human animal, wherein the non-human animal is a mouse, rat or pig.

As a finding concerning the maturing of hematopoietic cells and hepatocytes, reports have been made that a cytokine (OSM) produced by hematopoietic cells in the developmental process of mouse promotes the maturing of hepatocytes (Non-Patent Document No. 2: Kamiya A., et al., EMBO J. 1999 18:2127-3; Non-Patent Document No. 3: Kinoshita T., et al., Proc Natl Acad Sci U S A. 1999 96:7265-70 (1999)). However, in the conventional two-dimensional culture, directed differentiation was utterly insufficient though OSM had been already added. On the other hand, the patent of the present inventors (Patent Document No. 1: Method for Preparing Tissue and Organ WO2013/047639 A1), although it discloses preparation of organ buds from three types of cells, does not disclose or even suggest a methodology that enables organ buds to be formed from four or more types of cells including hematopoietic cells and which also promotes the maturing thereof. Therefore, the introduction of the fourth cell (hematopoietic cells) into *in vitro* reconstructed organ buds and the finding of its importance in the maturing of a functional cell of interest have great novelty and inventiveness.

### EFFECT OF THE INVENTION

To date, the present inventors have succeeded in constructing a platform technology for preparing a human tissue/organ in which a vascular system is appropriately located; such a human tissue/organ has never been achieved by conventional techniques (Method for Preparing Tissue and Organ). In order to substantially advance the above-described technology, the present invention has achieved a technique to greatly promote the maturing of a functional cell of interest through direct or indirect cellular action by adding a new player (i.e., hematopoietic cells). This is a completely novel technique that achieves maturing of metabolic and other *in vitro* functions that have heretofore been inadequate. Specifically, according to the technique of the present invention, hepatocytes were recognized to improve in function by a factor of about 3 to 4 (taking albumin secretion as an indicator). Accordingly, it is expected that an equivalent function will be exhibited by cells whose number is three to four times less than the conventionally needed. As a result, the production cost can be reduced to between one third and a fourth. In terms of the current treatment cost per adult terminal hepatic failure patient, it is expected that a reduction of approx. 6 million Yen will be achieved.

Further, as a result of the functional improvement according to the present invention, it is expected that drug metabolism and other functions that have heretofore been difficult to evaluate can be detected, which also enables application to *in vitro* screenings in drug discovery.

The present specification encompasses the contents disclosed in the specification and/or the drawings of Japanese Patent Application No. 2015-251140 based on which the present patent application claims priority.

### BRIEF DESCRIPTION OF THE DRAWINGS

[Fig. 1] Isolation of a cell fraction comprising hematopoietic cells from cord blood using a cell sorter.
[Fig. 2] Preparation of liver buds comprising blood cells (left panel); and measurement by ELISA of albumin secretion into medium from liver buds comprising blood cells (right panel).
[Fig. 3] Examination of genetic expression of hepatic differentiation markers by quantitative PCR in the preparation of liver buds comprising blood cells.
[Fig. 4] Examination of the amount of blood cells added in the preparation of liver buds comprising blood cells.
[Fig. 5] Albumin secretion dependent on the amount of blood cells added.

### BEST MODES FOR CARRYING OUT THE INVENTION

Hereinbelow, the present invention will be described in detail.

The present disclosure provides a method of preparing an organ bud, comprising culturing vascular endothelial cells, mesenchymal cells and a tissue or organ cell *in vitro* in the presence of blood cells.

As used herein, the term "organ bud" means a structure capable of differentiating into an organ through maturing, the structure comprising four types of cells, i.e., tissue or organ cells, vascular endothelial cells, mesenchymal cells (undifferentiated mesenchymal cells or cells differentiated therefrom) and blood cells. Whether a structure is an organ bud or not can be determined, for example, by transplanting the structure into an organism and examining whether or not it is capable of differentiating into an organ of interest (the structure can be judged as organ bud if it has differentiated into the organ of interest); and/or by examining whether or not the structure comprises all of the above-listed four types of cells (the structure can be judged as organ bud if it comprises all of the four types of cells). The organ bud may be one which differentiates into an organ such as kidney, heart, lung, spleen, esophagus, stomach, thyroid, parathyroid, thymus, gonad, brain, spinal cord or the like. Preferably, the organ bud is one which differentiates into an endodermal organ such as one which differentiates into liver (liver bud), one which differentiates into pancreas (pancreas bud), or one which differentiates into intestinal tract. Whether a certain structure is an organ bud which differentiates into an endodermal organ or not can be determined by examining the expression of marker proteins (if any one or more of the marker proteins described later are expressed, the organ bud can be judged as the organ bud of interest). For example, HHEX, SOX2, HNF4A, AFP, ALB and the like are markers for liver buds; PDX1, SOX17, SOX9 and the like are markers for pancreas bud; and CDX2, SOX9 and the like are markers for organ buds which differentiate into intestinal tract. Among the terms used by those skilled in the art, the following are included in the organ bud of the present disclosure: liver bud, liver diverticula, liver organoid, pancreatic (dorsal or ventral) buds, pancreatic diverticula, pancreatic organoid, intestinal bud, intestinal diverticula, intestinal organoid (K. Matsumoto, et al. Science.19; 294 (5542): 559-63 (2001)) and so on.

As used herein, the term "blood cell" means a cell isolated from a living body, a cell obtained from stem cells such as ES cells or iPS cells by directed differentiation, or a cell directly reprogrammed by gene transfer into a differentiated cell, on the condition that the following characteristics of blood cells are displayed (e.g., expressing any one of CD34, CD2, CD3, CD4, CD7, CD8, CD10, CD14, CD16, CD19, CD20, CD24, CD41, CD45, CD56, CD66b or CD235a, CD38, CD90, CD49f, VEGFR2, CD43, CD71, GPA (Glycophorin A), CD42b, c-kit, CD150, Sca-1, Ter119 or the like). Whether a cell is a blood cell or not may be determined by checking for the expression of CD34, CD2, CD3, CD4, CD7, CD8, CD10, CD14, CD16, CD19, CD20, CD24, CD41, CD45, CD56, CD66b or CD235a, CD38, CD90, CD49f, VEGFR2, CD43, CD71, GPA (Glycophorin A), CD42b, c-kit, CD150, Sca-1, Ter119 or the like. The blood cell used in the present invention may be either differentiated or undifferentiated. Preferably, the blood cell comprises undifferentiated hematopoietic cells such as hematopoietic progenitor cells or hematopoietic stem cells. As undifferentiated hematopoietic cells, blood cells derived from pluripotent stem cells such as induced pluripotent stem cells (iPS cells), embryonic stem cells (ES cells), etc.; blood cells collected from blood (cord blood, bone marrow blood, peripheral blood, etc.); or blood cells directly reprogrammed from other differentiated cells may be enumerated, for example. In the Example described later, it is believed that hematopoietic progenitor cells and hematopoietic stem cells could be enriched by collecting the monocyte fractions of cord blood. Among the terms used by those skilled in the art, the following are included in the blood cell in the present invention: hematopoietic stem cell, blood stem cell, hematopoietic progenitor cell, blood progenitor cell, myeloid progenitor cell, granulocyte/monocyte progenitor cell, granulocyte precursor (progenitor?) cell, granulocyte, myeloblast, promyelocyte, myeloid cell, metamyelocyte, stab cell, segmented cell (neutrophil), monocyte progenitor, monocyte, macrophage, histiocyte, Kupffer cell, alveolar macrophage, microglia, osteoclast, epithelioid cell, giant cell (Langhans giant cell, foreign body giant cell, Touton giant cell), dendritic cell, Langerhans cell, myelomonocyte, myeloblast, basophilic promyelocyte, basophilic myeloid cell, basophilic metamyelocyte, basophil, myeloblast, eosinophilic promyelocyte, eosinophilic myeloid cell, eosinophilic metamyelocyte, eosinophilic stab cell, eosinophilic segmented cell (eosinophil), megakaryoblast, megakaryocyte, erythroblast, reticulocyte, mast cell precursor, and so on. As blood cells, human-derived blood cells are mainly used. However, blood cells derived from non-human animals (e.g., animals used, for example, as experimental animals, pet animals, working animals, race horses or fighting dogs, more specifically, mouse, rat, rabbit, pig, dog, monkey, cattle, horse, sheep, chicken, shark, devilfish, ratfish, salmon, shrimp, crab or the like) may also be used.

As used herein, the term "vascular endothelial cell" means cells constituting vascular endothelium or cells capable of differentiating into such cells. Whether a certain cell is vascular endothelial cell or not can be determined by checking for the expression of marker proteins such as TIE2, VEGFR-1, VEGFR-2, VEGFR-3 and CD41 (if any one or more of the above-listed marker proteins are expressed, the cell can be judged as vascular endothelial cell). The vascular endothelial cell used in the present invention may be either differentiated or undifferentiated. Whether a vascular endothelial cell is a differentiated cell or not can be determined by means of CD31 and CD144. Among the terms used by those skilled in the art, the following are included in the "vascular endothelial cell" of the present invention: endothelial cells, umbilical vein endothelial cells, endothelial progenitor cells, endothelial precursor cells, vasculogenic progenitors, hemangioblast (HJ. Joo, et al. Blood. 25;118(8):2094-104 (2011)) and so on. Preferable vascular endothelial cells are those derived from umbilical vein. Vascular endothelial cells may be collected from blood vessels, or may be prepared from pluripotent stem cells such as induced pluripotent stem cells (iPS cells) or embryonic stem cells (ES cells) according to known methods. As vascular endothelial cells, human-derived cells are mainly used. However, vascular endothelial cells derived from non-human animals (e.g., animals used, for example, as experimental animals, pet animals, working animals, race horses or fighting dogs, more specifically, mouse, rat, rabbit, pig, dog, monkey, cattle, horse, sheep, chicken, shark, devilfish, ratfish, salmon, shrimp, crab or the like) may also be used.

As used herein, the term "mesenchymal cell" means connective tissue cells that are mainly located in mesoderm-derived connective tissues and which form support structures for cells that function in tissues. The "mesenchymal cell" is a concept that encompasses those cells which are destined to, but are yet to, differentiate into mesenchymal cells. Mesenchymal cells used in the present invention may be either differentiated or undifferentiated. Whether a certain cell is an undifferentiated mesenchymal cell or not may be determined by checking for the expression of marker proteins such as Stro-1, CD29, CD44, CD73, CD90, CD105, CD133, CD271 or Nestin (if any one or more of the above-listed marker proteins are expressed, the cell can be judged as undifferentiated mesenchymal cell). A mesenchymal cell in which none of the above-listed markers are expressed can be judged as a differentiated mesenchymal cell. Among the terms used by those skilled in the art, the following are included in the "mesenchymal cell" of the present invention: mesenchymal stem cells, mesenchymal progenitor cells, mesenchymal cells (R. Peters, et al. PLoS One. 30; 5(12):e15689 (2010)) and so on. Preferable mesenchymal cells are mesenchymal cells derived from bone marrow (especially, mesenchymal stem cells). Mesenchymal cells may be collected from tissues such as bone marrow, adipose tissue, placental tissue, umbilical tissue, dental pulp or the like, or may be prepared from pluripotent stem cells such as induced pluripotent stem cells (iPS cells) or embryonic stem cells (ES cells) according to known methods. As mesenchymal cells, human-derived cells are mainly used. However, mesenchymal cells derived from non-human animals (e.g., animals used, for example, as experimental animals, pet animals, working animals, race horses or fighting dogs, more specifically, mouse, rat, rabbit, pig, dog, monkey, cattle, horse, sheep, chicken, shark, devilfish, ratfish, salmon, shrimp, crab or the like) may also be used.

As used herein, the term "tissue or organ cell" means functional cells constituting tissues or organs, or undifferentiated cells which differentiate into functional cells. Examples of "undifferentiated tissue or organ cell" include, but are not limited to, cells capable of differentiating into an organ such as kidney, heart, lung, spleen, esophagus, stomach, thyroid, parathyroid, thymus, gonad, brain or spinal cord; cells capable of differentiating into an ectodermal organ such as brain, spinal cord, adrenal medulla, epidermis, hair/nail/dermal gland, sensory organ, peripheral nerve or lens; cells capable of differentiating into a mesodermal organ such as kidney, urinary duct, heart, blood, gonad, adrenal cortex, muscle, skeleton, dermis, connective tissue or mesothelium; and cells capable of differentiating into an endodermal organ such as liver, pancreas, intestinal tract, lung, thyroid, parathyroid or urinary tract. Whether or not a certain cell is capable of differentiating into an ectodermal organ, mesodermal organ or endodermal organ can be determined by checking for the expression of marker proteins (if any one or more of marker proteins are expressed, the cell can be judged as a cell capable of differentiating into an endodermal organ). For example, in cells capable of differentiating into liver, HHEX, SOX2, HNF4A, AFP, ALB and the like are markers; in cells capable of differentiating into pancreas, PDX1, SOX17, SOX9 and the like are markers; in cells capable of differentiating into intestinal tract, CDX2, SOX9 and the like are markers; in cells capable of differentiating into kidney, SIX2 and SALL1 are markers; in cells capable of differentiating into heart, NKX2-5, MYH6, ACTN2, MYL7 and HPPA are markers; in cells capable of differentiating into blood, C-KIT, SCA1, TER119 and HOXB4 are markers; and in cells capable of differentiating into brain or spinal cord, HNK1, AP2, NESTIN and the like are markers. Among the terms used by those skilled in the art, the following are included in the "undifferentiated tissue or organ cell" of the present disclosure: hepatoblast, hepatic progenitor cells, hepatic precursor cells, pancreatoblast, pancreatic progenitors, pancreatic progenitor cells, pancreatic precursor cells, endocrine precursors, intestinal progenitor cells, intestinal precursor cells, intermediate mesoderm, metanephric mesenchymal precursor cells, multipotent nephron progenitor, renal progenitor cells, cardiac mesoderm, cardiovascular progenitor cells, cardiac progenitor cells (JR. Spence, et al. Nature.; 470(7332): 105-9.(2011); Self, et al. EMBO J.; 25(21): 5214-5228.(2006); J. Zhang, et al. Circulation Research.; 104: e30-e41(2009); G. Lee, et al. Nature Biotechnology 25, 1468-1475 (2007)) and so on. Undifferentiated tissue or organ cells may be collected from tissues or organs, or may be prepared from pluripotent stem cells such as induced pluripotent stem cells (iPS cells) or embryonic stem cells (ES cells) according to known methods. Moreover, undifferentiated tissue or organ cells may be such cells as primitive gut endoderm cells (PGECs) (Japanese Patent No. 5777127) which are at an intermediate stage of differentiation from pluripotent stem cells (e.g., iPS cells) into tissues or organs. PGECs are capable of differentiating into hepatocytes, pancreatic cells and enterocytes (have high differentiation function), do not express markers associated with the malignancy of cancer (are highly safe), and may be prepared from iPS cells by directed differentiation without using feeder cells. Therefore, PGECs have the advantage of even allowing for clinical application. Furthermore, mass preparation of PGECs is possible to. PGECs may be prepared according to the method disclosed in Japanese Patent No. 5777127. Alternatively, pluripotent stem cells such as iPS cells may be cultured in activin-supplemented serum-free medium so that they are induced to endodermal cells that are positive to both CXCR4 and E-cadherin; or the endodermal cells thus obtained may be cultured for two days in the presence of added BMP4 and FGF2 to obtain CXCR4-negative, HNF4α-positive hepatic endodermal cell populations. To give further examples, organ cells capable of differentiating into liver may be prepared as previously described (K.Si-Taiyeb, et al. Hepatology, 51 (1): 297- 305(2010); T. Touboul, et al. Hepatology. 51 (5): 1754-65 (2010)); organ cells capable of differentiating into pancreas may be prepared as previously described (D. Zhang, et al. Cell Res.;19(4):429-38 (2009)); organ cells capable of differentiating into intestinal tract may be prepared as previously described (J. Cai, et al. J Mol Cell Biol.; 2(1):50-60 (2010); R. Spence, et al. Nature.; 470 (7332): 105-9 (2011)); cells capable of differentiating into heart may be prepared as previously described (J. Zhang, et al. Circulation Research.; 104: e30-e41(2009); and cells capable of differentiating into brain or spinal cord may be prepared as previously described (G. Lee, et al. Nature Biotechnology 25, 1468 - 1475 (2007)). Examples of "differentiated tissue or organ cell" include, but are not limited to, endocrine cells of pancreas, pancreatic duct epithelial cells of pancreas, hepatocytes of liver, epithelial cells of intestinal tract, tubular epithelial cells of kidney, podocytes of kidney, cardiomyocytes of heart, lymphocytes and granulocytes of blood, erythrocytes, neurons and glial cells of brain, and neurons and Schwann cells of spinal cord. As tissue or organ cells, human-derived cells are mainly used. However, tissue or organ cells derived from non-human animals (e.g., animals used, for example, as experimental animals, pet animals, working animals, race horses or fighting dogs, more specifically, mouse, rat, rabbit, pig, dog, monkey, cattle, horse, sheep, chicken, shark, devilfish, ratfish, salmon, shrimp, crab or the like) may also be used.

Culture ratios of four cell types in coculture are not particularly limited as long as they are within the range that enables the formation of organ buds. Preferable cell count ratio is 10 : 10-5 : 2-0.1 : 2-50 for tissue or organ cell : vascular endothelial cell : mesenchymal cell : blood cell. Organ buds of approx. 50 to 250 µm in size may be formed by coculturing approx. 250,000 tissue or organ cells, approx. 170,000 vascular endothelial cells, approx. 25,000 mesenchymal cells and approx. 100,000 blood cells.

The medium used for culture may be any medium that enables the formation of organ buds and examples that are preferably used include a medium for culturing vascular endothelial cells, a medium for culturing tissue or organ cells, and a mixture of these two media. As a medium for culturing vascular endothelial cells, any medium may be used but, preferably, a medium containing at least one of the following substances may be used: hEGF (recombinant human epidermal growth factor), VEGF (vascular endothelial growth factor), hydrocortisone, bFGF, ascorbic acid, IGF1, FBS, antibiotics (e.g., gentamycin or amphotericin B), heparin, L-glutamine, phenol red and BBE. Specific examples of media that may be used for culturing vascular endothelial cells include, but are not limited to, EGM-2 BulletKit (Lonza), EGM BulletKit (Lonza), VascuLife EnGS Comp Kit (LCT), Human Endothelial-SFM Basal Growth Medium (Invitrogen) and human microvascular endothelial cell growth medium (TOYOBO). As a medium for culturing tissue or organ cells, any medium may be used but in the case where the organ cell is a hepatocyte, a medium containing at least one of ascorbic acid, BSA-FAF, insulin, hydrocortisone and GA-1000 may preferably be used. As a medium for culturing hepatocytes, HCM BulletKit (Lonza) from which hEGF (recombinant human epidermal growth factor) has been removed or RPMI1640 (Sigma-Aldrich) to which 1% B27 Supplements (GIBCO) and 10 ng/mL hHGF (Sigma-Aldrich) have been added may typically be used. As regards the formation of human liver buds, a 1:1 mixture of GM BulletKit (Lonza) and HCM BulletKit (Lonza) from each of which hEGF has been removed and which are each supplemented with dexamethasone, oncostatin M and HGF has been found effective for maturation of liver buds.

Although scaffold materials need not be used for culturing cells, a mixture of four types of cells may advantageously be cultured on a gel-like support that allows mesenchymal cells to contract.

Contraction of mesenchymal cells may be confirmed, for example, by noting the formation of a 3D tissue morphologically (either under microscope or with the naked eye) or by showing that the tissue is strong enough to retain its shape as it is collected with a spatula or the like (Takebe et al. Nature 499 (7459), 481-484, 2013).

The support may advantageously be a gel-like substrate having an appropriate stiffness [e.g., a Young's modulus of 200 kPa of less (in the case of a Matrigel-coated gel of a flat shape); however, the appropriate stiffness of the support may vary depending on the coating and shape]. Examples of such substrates include, but are not limited to, hydrogels (such as acrylamide gel, gelatin and Matrigel). The stiffness of the support need not be uniform and may vary with the shape, size and quantity of a cell condensate of interest so that it can be provided with a spatial/temporal gradient or can be patterned. In the case where the stiffness of the support is uniform, it is preferably 100 kPa or less, more preferably 1-50 kPa. The gel-like support may be planar, or alternatively, the side on which culture is to be performed may have a U- or V-shaped cross section. If the side of the gel-like support on which culture is to be performed has a U- or V-shaped cross section, cells tend to gather on the culture surface and a cell condensate can advantageously be formed from a smaller number of cells and/or tissues. Moreover, the support may be modified chemically or physically. Examples of modifying substances include, but are not limited to, Matrigel, laminin, entactin, collagen, fibronectin and vitronectin.

One example of the gel-like culture support that is provided with a spatial gradient of stiffness is a gel-like culture support that is stiffer in the central part than in the peripheral part. The stiffness of the central part is appropriately 200 kPa or less and it suffices that the peripheral part is softer than the central part. Appropriate values for the stiffness of the central and peripheral parts of the substrate are variable with the coating and the shape. Another example of the gel-like culture support that is provided with a spatial gradient of stiffness is a gel-like culture support that is stiffer in the peripheral part than in the central part.

One example of the patterned, gel-like culture support is a gel-like culture support having one or more patterns in which the central part is stiffer than the peripheral part. The stiffness of the central part is appropriately, 200 kPa or less and it suffices that the peripheral part is softer than the central part. Appropriate values for the stiffness of the central and peripheral parts of the substrate are variable with the coating and the shape. Another example of the patterned, gel-like culture support is a gel-like culture support having one or more patterns in which the peripheral part is stiffer than the central part. The stiffness of the peripheral part is appropriately 200 kPa or less and it suffices that the central part is softer than the peripheral part. Appropriate values for the stiffness of the central and peripheral parts of the substrate are variable with the coating and the shape.

The temperature during culture is not particularly limited but it is preferably 30-40°C and more preferably 37°C.

The culture period is not particularly limited but it is preferably 3-10 days and more preferably 6 days.

The organ bud prepared by the method of the present disclosure may be improved in function compared with an organ bud prepared by culturing vascular endothelial cells, mesenchymal cells and a tissue or organ cell *in vitro* in the absence of blood cells.

Consider, for example, the case where the organ bud is a liver bud; the liver bud prepared by the method of the present invention may have an improved albumin secretion capacity compared with a liver bud prepared by culturing vascular endothelial cells, mesenchymal cells and a tissue or organ cell *in vitro* in the absence of blood cells. Moreover, the liver bud prepared by the method of the present invention may have increased expression of hepatocyte differentiation marker genes compared with a liver bud prepared by culturing vascular endothelial cells, mesenchymal cells and a tissue or organ cell *in vitro* in the absence of blood cells. Examples of hepatocyte differentiation marker genes include, but are not limited to, α fetoprotein, albumin, CYP3A7, tryptophan metabolic enzyme TDO2 and sodium-taurocholate cotransporter.

The present disclosure also provides an organ bud prepared by the above-described method.

The thus prepared organ bud may be transplanted into a non-human animal, in which it is allowed to mature, whereby a tissue or organ can be prepared. Briefly, the present disclosure also provides, not part of the invention, a method of preparing a tissue or an organ, comprising transplanting the organ bud prepared by the above-described method into a non-human animal and differentiating the organ bud into a tissue or an organ. Examples of the non-human animal that may be used include animals that are used, for example, as experimental animals, pet animals, working animals, race horses or fighting dogs, more specifically, mouse, rat, rabbit, pig, dog, monkey, cattle, horse, sheep, chicken, shark, devilfish, ratfish, salmon, shrimp, crab and the like. Moreover, the non-human animal to be used herein is preferably an immunodeficient animal in order avoid immunorejection.

Therefore, the present disclosure also provides, not part of the invention, a method of transplanting an organ bud, comprising transplanting the organ bud prepared by the above-described method into a human or a non-human animal. The site of transplantation of the organ bud may be any site as long as transplantation is possible. Specific examples of the transplantation site include, but are not limited to, the intracranial space, the mesentery, the liver, the spleen, the kidney, the kidney subcapsular space, and the supraportal space. For intracranial transplantation, about 1 to 3 organ buds of 5 mm in size, prepared *in vitro,* may be transplanted. For intramesenteric transplantation, about 1 to 6 organ buds of 5 mm in size, prepared *in vitro,* may be transplanted. For transplantation into the supraportal space, about 1 to 20 organ buds of 5 mm in size, prepared *in vitro,* may be transplanted. For transplantation into the kidney subcapsular space, about 1 to 5 organ buds of 5 mm in size, prepared *in vitro,* may be transplanted. For transplantation into the liver, spleen or kidney, about 100 to 200 organ buds of 100 µm in size, prepared *in vitro,* may be transplanted.

The thus prepared tissue or organ may be used in drug discovery screening or regenerative medicine.

Therefore, the present disclosure provides a method of regeneration or function recovery of a tissue or an organ, comprising transplanting the organ bud prepared by the above-described method into a human or a non-human animal and differentiating the organ bud into a tissue or an organ. As non-human animals, animals used for such purposes as experimental animal, pet animal, working animal, race horse or fighting dog, more specifically, mouse, rat, rabbit, pig, dog, monkey, cattle, horse, sheep, chicken, shark, devilfish, ratfish, salmon, shrimp, crab or the like may be used.

The organ bud prepared by the method of the present disclosure may be formulated and used in the form of a composition for regenerative medicine. This composition of the present disclosure may be transplanted into a living body to prepare a tissue or an organ. Regeneration or function recovery of a tissue or an organ is also possible by transplanting the composition of the present disclosure into a living body.

Upon transplantation of the composition of the present disclosure into a living body, the organ bud may differentiate into a tissue or an organ with vascular networks. In such vascular networks, blood perfusion may occur. It is believed that the occurrence of blood perfusion in vascular networks enables generation of a tissue or an organ with a highly ordered tissue structure equivalent or close to the tissue structure of adult tissues.

The composition of the present disclosure may comprise a tissue vascularization promoter such as FGF2, HGF or VEGF, a gelatin sponge for hemostasis to cope with the bleeding from transplantation (product name: Spongel; Astellas Pharma), and a tissue adhesive for fixing transplants such as Bolheal (Teijin Pharma), Beriplast^{™} (CSL Behring) or TachoComb^{™} (CSL Behring).

The present disclosure also provides a method of preparing a non-human chimeric animal, comprising transplanting the organ bud prepared by the above-described method into a non-human animal and differentiating the organ bud into a tissue or an organ, wherein the non-human animal is a mouse, rat or pig. The non-human animal (e.g., mouse) transplanted with the organ bud may mimic the physiological function of the organismal species (e.g., human) from which the tissue or organ cell used for preparing the organ bud is derived.

Still further, the present disclosure provides a method of evaluating a drug, comprising using at least one member selected from the group consisting of the organ bud, the tissue or organ and the non-human chimeric animal as prepared by the above-described methods, respectively. Specific examples of drug evaluation include, but are not limited to, evaluation of drug metabolism (e.g., prediction of drug metabolism profiles), evaluation of drug efficacy (e.g., screening for drugs that are effective as pharmaceuticals), toxicity evaluation, and evaluation of drug interactions.

Evaluation of drug metabolism may be performed as follows. Briefly, is at least one member selected from the group consisting of the organ bud, the tissue or organ and the non-human chimeric animal as prepared by the above-described methods, respectively, is administered with a candidate compound for pharmaceuticals and the resulting biological sample is then collected and analyzed, whereby a human-type drug metabolism profile can be obtained. As a result, prediction of the distribution/metabolism/excretion processes of pharmaceuticals in human (which was extremely difficult to achieve by conventional methods) becomes possible and it is expected that the development of safe and efficacious pharmaceuticals can be greatly accelerated.

Screening for drugs that are effective as pharmaceuticals may be performed as follows. Briefly, at least one member selected from the group consisting of the organ bud, the tissue or organ and the non-human chimeric animal as prepared from a cell established from a diseased patient by the above-described methods, respectively, is administered with a novel candidate compound for pharmaceuticals. This enables subsequent analysis. As a result, a potential is expected for achieving great improvement in the precision of drug efficacy prediction for the case of actual administration to human, which has been unsatisfactory in conventional *in vitro* tests.

Evaluation of toxicity may be performed as follows. Briefly, at least one member selected from the group consisting of the organ bud, the tissue or organ and the non-human chimeric animal as prepared by the above-described methods, respectively, is administered with a test substance and, thereafter, histological damage markers or the like are measured. This makes it possible to improve the precision of damage prediction.

Evaluation of drug interactions may be performed as follows. Briefly, at least one member selected from the group consisting of the organ bud, the tissue or organ and the non-human chimeric animal as prepared by the above-described methods, respectively, is administered with a plurality of drugs; then, each drug is examined for its pharmacokinetics such as distribution/metabolism/excretion processes, evaluated for its toxicity, and evaluated for its efficacy.

Further, it is also possible to create tissue stem cells from the tissue or organ prepared by the method of the present invention. Thus, the present invention is applicable to cell engineering techniques for large scale creation of human tissue cells and organ cells.

### EXAMPLES

Hereinbelow, the present invention will be described in more detail with reference to the following Example.

### [Example 1]

### [Methods]

### 1. Preparation of Blood Cells

### <Acquisition of Cord Blood>

Blood was collected from the umbilical cord (kindly supplied by a patient who gave birth to a child by Caesarean section in the Yokohama City University Hospital) with a 50 ml syringe and an 18G needle. Approx. 40 to 50 ml of blood was collected.

### <Lysis Treatment>

Lysis buffer (40 ml) was placed in a 50 ml tube, to which 10 ml of the cord blood was added.

After stirring, the tube was left stationary for 10 min. at room temperature.

The tube was centrifuged at 200G for 10 min.

The resultant precipitate was re-suspended in 10 ml of lysis buffer. The contents of five tubes were collected together to make a 50 ml suspension.

The above suspension was re-centrifuged at 200G for 10 min.

The precipitate was suspended in 10 ml of DMEM+10% FBS and centrifuged at 200G for 5 min.

The precipitate was re-suspended in 10 ml of DMEM+10% FBS, followed by cell counting.

### Materials

### 1) Preparation of 10 × Lysis Buffer (×10 liquid; 1/10 dilution was used.)

▪ NH₄Cl 82.6 g
▪ NaHCO₃ 11.9 g
▪ EDTA₂Na 0.378 g
/1L milliQ adjusted to pH 7.3

### 2) Preparation of 1 × Lysis Buffer

Dilution (×1) with PBS (preferably cooled to 4°C; 2 mM EDTA may be added) was used.

### <Isolation of PI⁻/CD235⁻ Cells>

Dead cells were stained with propidium iodide (PI); erythrocytes/erythroblasts were stained with fluorescence-labeled CD235 antibody; cells not stained with either PI or the antibody (viable cells and neither erythrocytes nor erythroblasts) were isolated with a cell sorter.

### <Isolation of Monocyte Fraction (MNC) Cells>

Using a cell sorter, PI⁻/CD235⁻ cells were developed with FSC (forward scatter) and SSC (side scatter) to isolate cell populations classified as monocyte fraction (MNC) cells.

### 2. Preparation of Hepatic Endoderm Cells

### Method:

Undifferentiated iPS cells (TkDA3 supplied by the University of Tokyo and an iPS cell line established from umbilical cord by the present inventors) were exfoliated to prepare single cells. The cells were cultured in plastic dishes at a density of 5×10⁴ cells/cm² in the presence of RPMI+1% B27+10 uM Rockinhibitor+50 ng/mL Wnt3a+100 ng/mL Activin A for one day. Subsequently, the resultant cells were cultured in the presence of RPMI+1% B27+50 ng/mL Wnt3a+100 ng/mL Activin A for six days to thereby obtain hepatic endoderm cells.

### [Results]

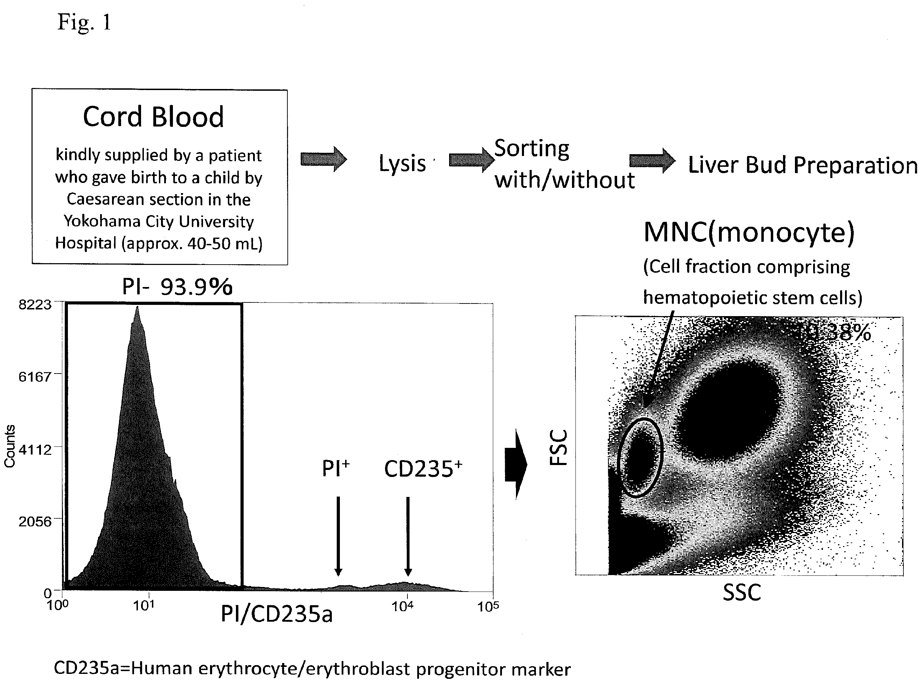

### 1. (Figs. 1, 2 and 3)

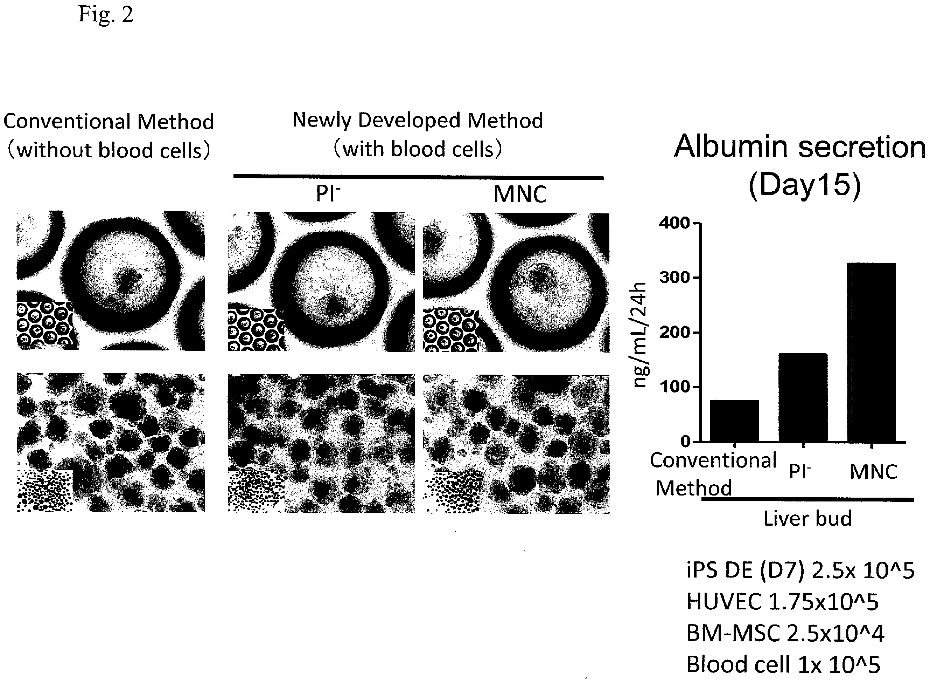

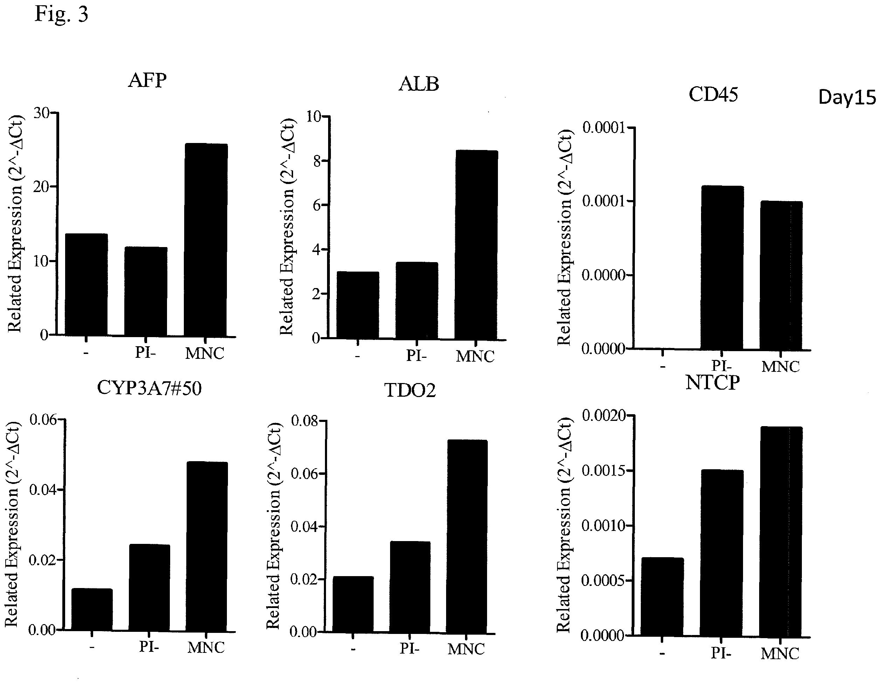

In a 10:7:1 mixture of hepatic endoderm cells (2.5×10⁵ cells), umbilical cord-derived vascular endothelial cells (1.7×10⁵ cells) (Lonza, Basel, Switzerland) and mesenchymal cells (2.5×10⁴ cells) (Lonza, Basel, Switzerland), a cell sorter isolated PI⁻cells and monocyte fraction (MNC) cells (Fig. 1) enriched in hematopoietic stem cells/hematopoietic progenitor cells were suspended (1×10⁵ cells for each), followed by preparation of liver buds (50-250 µm in size) on Kuraray microwell plates. After 15 days culture, protein amounts of human albumin secreted in 24 hr into the culture supernatants of liver buds cultured under respective conditions were analyzed by ELISA. As a result, the groups to which PI⁻ cells or hematopoietic stem cells/hematopoietic progenitor cells-enriched monocyte fraction (MNC) cells were added showed a greater amount of albumin secretion than the group to which no blood cells were added (conventional method) (Fig. 2). Subsequently, the liver buds at day 15 of culture under the above-described conditions were collected, followed by checking for the expression of hepatocyte differentiation markers (α fetoprotein (AFP), albumin (ALB), CYP3A7, tryptophan metabolic enzyme (TDO2) and sodium-taurocholate cotransporter (NTCP)) by qPCR. As a result, the groups to which PI⁻ cells or monocyte fraction (MNC) cells were added showed higher gene expression levels than the groups to which no blood cells were added (conventional method) (Fig. 3).

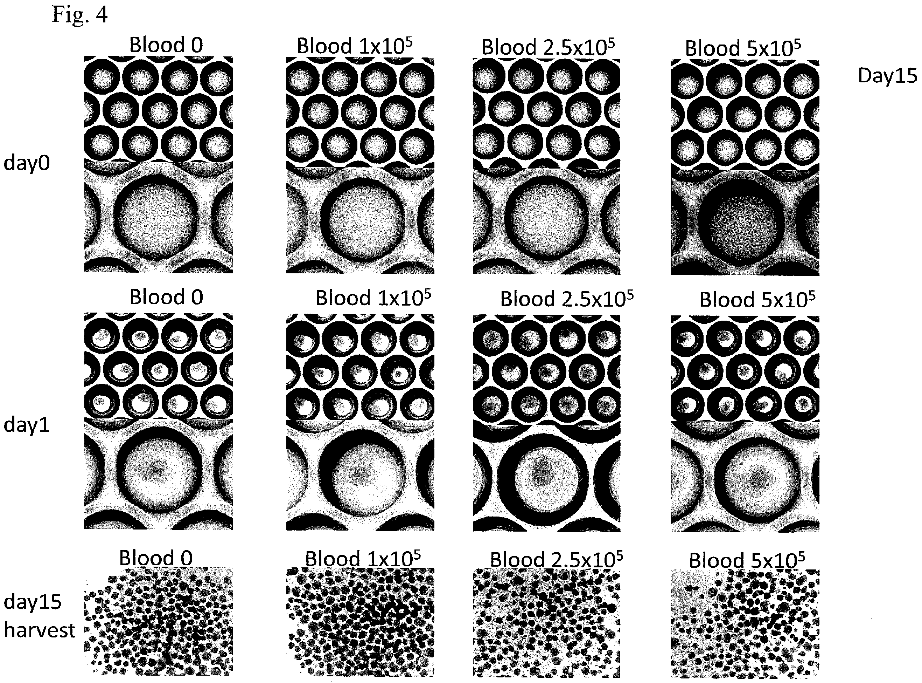

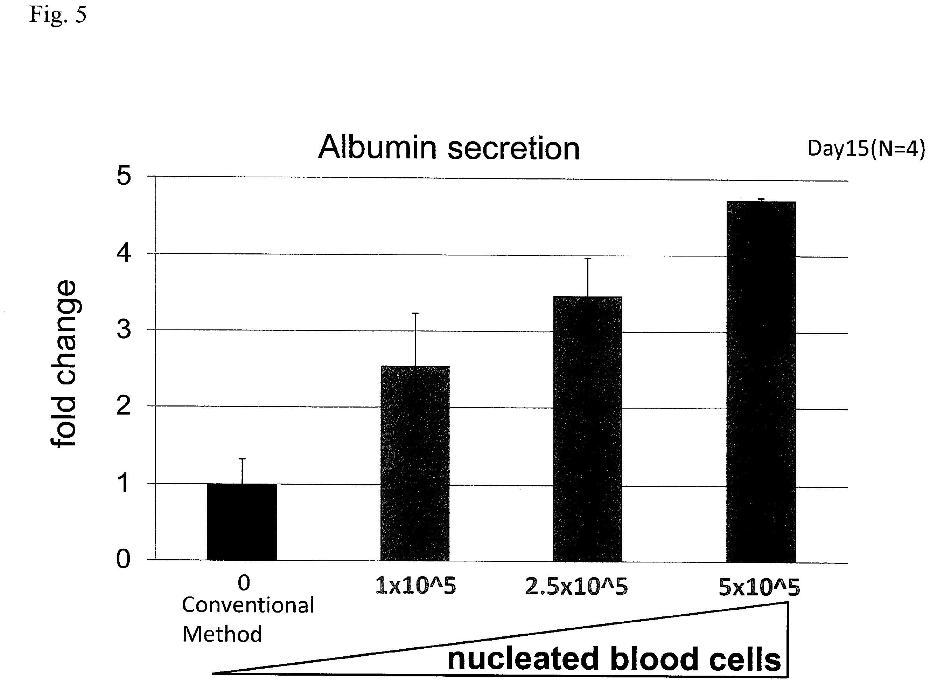

### 2. (Figs. 4 and 5)

In order to examine the dose dependency of blood cells, experimental groups were prepared as follows. To a 10:7:1 cell mixture of hepatic endoderm cells (2.5×10⁵ cells), umbilical cord-derived vascular endothelial cells (1.7×10⁵ cells) and mesenchymal cells (2.5×10⁴ cells), no blood cells were added or blood cells (PI⁻ cells) were added at varying densities of 1×10⁵ cells, 2.5×10⁵ cells or 5×10⁵ cells. At day 15 of liver bud preparation, the amount of human albumin secretion in 24 hr in culture broth (Fig. 4) was examined. The results revealed that the protein amount of human albumin secretion was enhanced in a manner dependent on blood cell count, as compared with the conventional method (Fig. 5).

### INDUSTRIAL APPLICABILITY

The present invention will provide an important platform technology directed to industrial production of human functional cells. The invention is applicable to preparation of human tissues/organs for transplantation as a technology of regeneration medicine targeting refractory diseases. Not only a great cost reduction is expected compared with conventional methods, but also improvement in functional maturation *in vitro* might be possible with the technique of the present invention. Therefore, the present invention is expected to become applicable to even acute/subacute hepatic failure which has been difficult to treat by the conventional technology.

Further, a drug evaluation system established using a human hepatic tissue artificially prepared according to the present invention would enable large scale production of human mature hepatocytes needed in drug discovery/development.

## Claims

1. A method of preparing a liver bud, comprising culturing vascular endothelial cells, mesenchymal cells, a cell capable of differentiating into a liver cell, and blood cells, wherein the blood cells are derived from cord blood, *in vitro.*

2. The method of claim 1, wherein the liver bud is a structure capable of differentiating into a liver through maturing.

3. The method of claim 1 or 2, wherein cells are cultured without using scaffold materials.

4. The method of any one of claims 1 to 3, wherein the blood cell comprises an undifferentiated hematopoietic cell, and optionally wherein the undifferentiated hematopoietic cell is a hematopoietic progenitor cell and/or a hematopoietic stem cell.

5. The method of any one of claims 1 to 4, wherein the blood cell derived from cord blood is a cell of the monocyte fraction of cord blood.

6. The method of any one of claims 1 to 5, wherein the cell capable of differentiating into a liver cell is: (i) an undifferentiated cell induced from a pluripotent stem cell, and optionally wherein the pluripotent stem cell is an induced pluripotent stem cell; or (ii) a hepatic endoderm cell.

7. The method of claim 6, wherein the pluripotent stem cell is derived from human.

8. The method of any one of claims 1 to 7, wherein a liver bud with an improved albumin secretory capacity is prepared compared with a liver bud prepared by culturing vascular endothelial cells, mesenchymal cells and a cell capable of differentiating into a liver cell *in vitro* in the absence of blood cells.

9. The method of claim 8, wherein a liver bud with increased expression of hepatocyte differentiation marker genes is prepared compared with a liver bud prepared by culturing vascular endothelial cells, mesenchymal cells and a cell capable of differentiating into a liver cell *in vitro* in the absence of blood cells.

10. The method of claim 9, wherein the hepatocyte differentiation marker gene is at least one marker selected from the group consisting of α fetoprotein, albumin, CYP3A7, tryptophan metabolic enzyme TDO2 and sodium-taurocholate cotransporter.

11. A liver bud prepared by the method of any one of claims 1 to 10.

12. A method of preparing a human liver, comprising preparing a human liver bud by the method of any one of claims 1 to 10, wherein the liver bud is capable of differentiating into a human liver after the liver bud has been transplanted into a non-human animal.

13. The liver bud of claim 11 for use in a therapeutic method comprising transplanting the liver bud into a human or a non-human animal in need thereof.

14. The liver bud of claim 11 for use in a therapeutic method of regeneration or function recovery of a liver, comprising transplanting the liver bud into a human or a non-human animal and differentiating the liver bud into a liver.

15. A method of preparing a non-human chimeric animal, comprising preparing a human liver bud by the method of any of claims 1 to 10, wherein the liver bud is capable of differentiating into a human liver after it has been transplanted into a non-human animal, wherein the non-human animal is a mouse, rat or pig.

## Patentansprüche

1. Verfahren des Herstellens einer Leberknospe, umfassend das Kultivieren von vaskulären Endothelzellen, mesenchymalen Zellen, einer Zelle, die in eine Leberzelle differenzieren kann, und Blutzellen, wobei die Blutzellen von Nabelschnurblut *in vitro* abgeleitet sind.

2. Verfahren nach Anspruch 1, wobei die Leberknospe eine Struktur ist, die in eine Leber durch Reifung differenzieren kann.

3. Verfahren nach Anspruch 1 oder 2, wobei Zellen ohne Verwendung von Scaffoldmaterialien kultiviert werden.

4. Verfahren nach einem der Ansprüche 1 bis 3, wobei die Blutzelle eine undifferenzierte hämatopoetische Zelle umfasst, und wobei optional die undifferenzierte hämatopoetische Zelle eine hämatopoetische Progenitorzelle und/oder hämatopoetische Stammzelle ist.

5. Verfahren nach einem der Ansprüche 1 bis 4, wobei die Blutzelle, die von Nabelschnurblut abgeleitet ist, eine Zelle der Monozytenfraktion von Nabelschnurblut ist.

6. Verfahren nach einem der Ansprüche 1 bis 5, wobei die Zelle, die in der Lage ist, in eine Leberzelle zu differenzieren, Folgendes ist: (i) eine undifferenzierte Zelle, induziert aus einer pluripotenten Stammzelle, und wobei optional die pluripotente Stammzelle eine induzierte pluripotente Stammzelle ist; oder (ii) eine hepatische Entodermzelle.

7. Verfahren nach Anspruch 6, wobei die pluripotente Stammzelle von Menschen abgeleitet ist.

8. Verfahren nach einem der Ansprüche 1 bis 7, wobei eine Leberknospe mit einer verbesserten Albumin-Sekretionsleistung hergestellt wird im Vergleich zu einer Leberknospe, die hergestellt wird durch Kultivieren von vaskulären Endothelzellen, mesenchymalen Zellen und einer Zelle, die in eine Leberzelle in Abwesenheit von Blutzellen *in vitro* differenzieren kann.

9. Verfahren nach Anspruch 8, wobei eine Leberknospe mit erhöhter Expression von Markergenen für hepatozytäre Differenzierung hergestellt wird im Vergleich zu einer Leberknospe, die hergestellt wird durch Kultivieren von vaskulären Endothelzellen, mesenchymalen Zellen und einer Zelle, die in eine Leberzelle in Abwesenheit von Blutzellen *in vitro* differenzieren kann.

10. Verfahren nach Anspruch 9, wobei das Markergen für hepatozytäre Differenzierung mindestens ein Marker ist, ausgewählt aus der Gruppe bestehend aus α-Fetoprotein, Albumin, CYP3A7, Tryptophan-Stoffwechselenzym TDO2 und Natrium-Taurocholat-Cotransporter.

11. Leberknospe, hergestellt durch das Verfahren nach einem der Ansprüche 1 bis 10.

12. Verfahren des Herstellens einer menschlichen Leber, umfassend das Herstellen einer menschlichen Leberknospe durch das Verfahren nach einem der Ansprüche 1 bis 10, wobei die Leberknospe in eine menschliche Leber differenzieren kann, nachdem die Leberknospe in ein nicht-menschliches Tier transplantiert wurde.

13. Leberknospe nach Anspruch 11 zur Verwendung in einem therapeutischen Verfahren, umfassend das Transplantieren der Leberknospe in einen Menschen oder ein nicht-menschliches Tier, der/das dessen bedarf.

14. Leberknospe nach Anspruch 11 zur Verwendung in einem therapeutischen Verfahren der Regenerierung oder Funktionswiederherstellung einer Leber, umfassend das Transplantieren der Leberknospe in einen Menschen oder ein nicht-menschliches Tier und Differenzieren der Leberknospe in eine Leber.

15. Verfahren des Herstellens eines nicht-menschlichen chimären Tiers, umfassend das Herstellen einer menschlichen Leberknospe durch das Verfahren nach einem der Ansprüche 1 bis 10, wobei die Leberknospe in eine menschliche Leber differenzieren kann, nachdem sie in ein nicht-menschliches Tier transplantiert wurde, wobei das nicht-menschliche Tier eine Maus, eine Ratte oder ein Schwein ist.

## Revendications

1. Procédé de préparation d'un bourgeon hépatique, comprenant la culture de cellules endothéliales vasculaires, de cellules mésenchymateuses, d'une cellule capable de se différencier en une cellule hépatique, et de cellules sanguines, les cellules sanguines étant issues du sang de cordon, *in vitro.*

2. Procédé selon la revendication 1, dans lequel le bourgeon hépatique est une structure capable de se différencier en un foie par maturation.

3. Procédé selon la revendication 1 ou 2, dans lequel les cellules sont cultivées sans utiliser de matériaux d'échafaudage.

4. Procédé selon l'une quelconque des revendications 1 à 3, dans lequel la cellule sanguine comprend une cellule hématopoïétique indifférenciée, et éventuellement dans lequel la cellule hématopoïétique indifférenciée est une cellule progénitrice hématopoïétique et/ou une cellule souche hématopoïétique.

5. Procédé selon l'une quelconque des revendications 1 à 4, dans lequel la cellule sanguine issue du sang de cordon est une cellule de la fraction monocyte du sang de cordon.

6. Procédé selon l'une quelconque des revendications 1 à 5, dans lequel la cellule capable de se différencier en une cellule hépatique est : (i) une cellule indifférenciée induite à partir d'une cellule souche pluripotente, et éventuellement dans lequel la cellule souche pluripotente est une cellule souche pluripotente induite ; ou (ii) une cellule d'endoderme hépatique.

7. Procédé selon la revendication 6, dans lequel la cellule souche pluripotente est d'origine humaine.

8. Procédé selon l'une quelconque des revendications 1 à 7, dans lequel un bourgeon hépatique avec une capacité de sécrétion d'albumine améliorée est préparé par rapport à un bourgeon hépatique préparé par culture de cellules endothéliales vasculaires, de cellules mésenchymateuses et d'une cellule capable de se différencier en une cellule hépatique *in vitro* en l'absence de cellules sanguines.

9. Procédé selon la revendication 8, dans lequel un bourgeon hépatique avec une expression accrue de gènes marqueurs de différenciation hépatocytaire est préparé par rapport à un bourgeon hépatique préparé par culture de cellules endothéliales vasculaires, de cellules mésenchymateuses et d'une cellule capable de se différencier en une cellule hépatique *in vitro* en l'absence de cellules sanguines.

10. Procédé selon la revendication 9, dans lequel le gène marqueur de différenciation hépatocytaire est au moins un marqueur choisi dans le groupe constitué par α-foetoprotéine, l'albumine, CYP3A7, l'enzyme métabolique tryptophane TDO2 et le cotransporteur taurocholate de sodium.

11. Bourgeon de foie préparé par le procédé de l'une quelconque des revendications 1 à 10.

12. Procédé de préparation d'un foie humain, comprenant la préparation d'un bourgeon hépatique humain par le procédé de l'une quelconque des revendications 1 à 10, dans lequel le bourgeon hépatique est capable de se différencier en un foie humain après que le bourgeon hépatique a été transplanté dans un animal non humain.

13. Bourgeon hépatique selon la revendication 11 pour une utilisation dans un procédé thérapeutique comprenant la transplantation du bourgeon hépatique chez un humain ou un animal non humain en ayant besoin.

14. Bourgeon hépatique selon la revendication 11 pour une utilisation dans un procédé thérapeutique de régénération ou de récupération de fonction d'un foie, comprenant la transplantation du bourgeon hépatique chez un humain ou un animal non humain et la différenciation du bourgeon hépatique en un foie.

15. Procédé de préparation d'un animal chimère non humain, comprenant la préparation d'un bourgeon hépatique humain par le procédé de l'une quelconque des revendications 1 à 10, dans lequel le bourgeon hépatique est capable de se différencier en un foie humain après avoir été transplanté dans un animal non humain, dans lequel l'animal non humain est une souris, un rat ou un porc.
